Europäisches Patentamt

European Patent Office

Office européen des brevets

(19)

(11) **EP 1 284 637 B1**

(12) **EUROPÄISCHE PATENTSCHRIFT**

(45) Veröffentlichungstag und Bekanntmachung des Hinweises auf die Patenterteilung:
**23.06.2004 Patentblatt 2004/26**

(51) Int Cl.⁷: **A61B 3/10**

(21) Anmeldenummer: **01916837.6**

(86) Internationale Anmeldenummer:
**PCT/CH2001/000226**

(22) Anmeldetag: **09.04.2001**

(87) Internationale Veröffentlichungsnummer:
**WO 2002/080760 (17.10.2002 Gazette 2002/42)**

(54) **VERFAHREN UND VORRICHTUNGSANORDNUNG ZUR BESTIMMUNG DER HORNHAUTDICKE EINES AUGES**

METHOD AND DEVICE FOR DETERMINING THE CORNEA THICKNESS OF AN EYE

PROCEDE ET DISPOSITIF POUR DETERMINER L'EPAISSEUR DE LA CORNEE D'UN OEIL

(84) Benannte Vertragsstaaten:
**AT BE CH CY DE DK ES FI FR GB GR IE IT LI LU MC NL PT SE TR**

(43) Veröffentlichungstag der Anmeldung:
**26.02.2003 Patentblatt 2003/09**

(73) Patentinhaber: **SIS AG, Surgical Instruments Systems**
**2555 Brügg b. Biel (CH)**

(72) Erfinder: **RATHJEN, Christian**
**28197 Bremen (DE)**

(74) Vertreter: **Vogel, Dany et al**
**Isler & Pedrazzini AG**
**Patentanwälte**
**Postfach 6940**
**8023 Zürich (CH)**

(56) Entgegenhaltungen:
**DE-U- 29 612 466**          **US-A- 5 512 965**

- **HITZENBERGER C K: "MEASUREMENT OF CORNEAL THICKNESS BY LOW-COHERENCE INTERFEROMETRY" APPLIED OPTICS, OPTICAL SOCIETY OF AMERICA,WASHINGTON, US, Bd. 31, Nr. 31, 1. November 1992 (1992-11-01), Seiten 6637-6642, XP000310747 ISSN: 0003-6935**
- **MUTTI ET AL: 'Artifact in optical pachometry with contact lenses in situ' AMERICAN JOURNAL OF OPTOMETRY&PHYSIOLOGICAL OPTICS Bd. 63, Nr. 10, 1986, Seiten 847 - 852**

**Beschreibung**

Die vorliegende Erfindung betrifft ein Verfahren und eine Vorrichtungsanordnung zur Bestimmung der Hornhautdicke eines Auges. Insbesondere betrifft die vorliegende Erfindung ein Verfahren und eine Vorrichtungsanordnung zur Bestimmung der Hornhautdicke eines menschlichen Auges, mittels Lichtwellen, wobei erste Lichtwellen auf und in die Hornhaut gestrahlt werden und wobei von der Hornhaut reflektierte zweite Lichtwellen zur Bestimmung der Hornhautdicke erfasst werden.

Neben der Verwendung von Ultraschall zur Bestimmung einer über einen grossen Bereich gemittelten Hornhautdicke eines menschlichen Auges werden gegenwärtig Aufsätze für Spaltlampen zur Pachymetrie eingesetzt, um die Hornhautdicke mittels Lichtwellen zu bestimmen. Ein solcher Aufsatz erzeugt mittels einer drehbaren planparallelen Platte im Beobachtungsstrahlengang eines Spaltlampenmikroskops zwei versetzte Halbbilder des Lichtschnitts mit der Hornhaut. Durch Drehen der planparallelen Platte können die versetzten Lichtschnitte zur Deckung gebracht werden und ein der Drehung entsprechender Messwert für die Hornhautdicke abgelesen werden. Eine solche Messung findet nur an einem Punkt der Hornhaut statt, erfordert manuelles Geschick und ist schwer reproduzierbar, da die Lage des Messpunkts nicht definiert ist. Der Nachteil dieser Verfahren zur Bestimmung der Hornhautdicke liegt insbesondere darin, dass sie sich nicht für eine Bestimmung der Hornhautdicke mit einer hohen Ortsauflösung eignen, das heisst, dass sie keine flächige oder flächendeckende Bestimmung der Hornhautdicke ermöglichen. Folglich können lokale Abweichungen der Hornhautdicke durch diese Verfahren nicht erfasst werden, was insbesondere bei chirurgischen Eingriffen in der Ophtalmologie (z.B. refraktive Chirurgie) äusserst risikoreich und daher nachteilig ist.

In der Patentschrift US 5512965 werden eine Vorrichtung und ein Verfahren beschrieben, die auf einer modifizierten Spaltlampe basieren und mittels welchen ein dreidimensionaler Ausdruck der Hornhautoberfläche und der jeweiligen lokalen Hornhautdicke erzeugt werden kann. Die Vorrichtung gemäss US 5512965 umfasst eine modifizierte Spaltlampe, deren Projektionsschlitz zur Verbesserung der Tiefenschärfe gebogen ist, eine Fernsehkamera mit dazugehörendem Linsensystem, elektronische Schaltkreise zur Auswahl und Quantifizierung von aufgenommenen Fernsehbildern, sowie ein Steuermechanismus zur Bewegung des durch die Spaltlampe erzeugten Lichtspalts. Gemäss US 5512965 werden mehrere digital codierte Bilder der erfassten Lichtschnitte mittels Software ausgewertet, wobei Messungen einzelner Lichtschnitte über Referenzmarken des Auges (Limbus und Purkinje Bilder der Austrittspupille und Glanzlichter des Spaltprojektors) zur Gesamtmessung zusammengesetzt werden. Gemäss dem in US 5512965 beschriebenen Verfahren muss der Patient mit dem zu vermessenden Auge ein Zielobjekt fixieren, damit zu grosse Bewegungen des Auges während der Messung vermieden werden. Gemäss US 5512965 wird der Fokus der Fernsehkamera während der Messung mit einem zusätzlichen Aufwand der Hornhaut nachgeführt. Da die Brechung der in die Hornhaut einfallenden Strahlen der Lichtschnitte bekannterweise von der lokalen Oberflächenneigung der Hornhaut abhängt, muss im Verfahren gemäss US 5512965 zur Bestimmung der Hornhautdicke die Neigung der Hornhaut mit einem zusätzlichen Messaufwand gemessen werden. Das Verfahren gemäss US 5512965 erfordert zudem bei jeder Messung eine aufwendige Kalibrierung, da sich der Beleuchtungswinkel, der Beobachtungswinkel sowie der Objektabstand dauernd ändern. Das Verfahren gemäss US 5512965 erfordert überdies eine aufwendige Ausrichtung des Patienten mit zusätzlichen Marken. Bedingt durch den hohen gerätetechnischen Aufwand, wird das Verfahren gemäss US 5512965 nur in Form eines Standgeräts ausgeführt, was den Nachteil hat, dass das Verfahren nicht bei liegenden Patienten, beispielsweise im Operationssaal, einsetzbar ist.

Die Bedeutung einer flächigen Hornhautdickenmessung hoher Auflösung in der Ophtalmologie wird besonders bei chirurgischen Eingriffen an der Hornhaut ersichtlich. Insbesondere dort, wo die Hornhaut in definierter Tiefe geschnitten wird oder wo in die Hornhaut in definierter Tiefe eingedrungen wird, ist eine genaue Kenntnis der örtlichen Hornhautdicke wichtig. Beispiele solcher chirurgischen Eingriffe sind die radiale Keratotomie, LASIK (Laser In Situ Keratomileusis) und ALK (Automated Lamellar Keratoplasty). Während bei der radialen Keratotomie dünne oberflächliche Schlitze in die Hornhaut geschnitten werden, werden bei LASIK und ALK im applanierten Zustand der Hornhaut dünne Schichten von der Hornhaut geschnitten. Aus medizinischer Sicht ist es daher äusserst wichtig, vor dem operativen Eingriff die aktuelle Hornhautdicke zu kennen. Erst mit der Kenntnis der Hornhautdicke lässt sich sicherstellen, dass nicht zu tief geschnitten wird und keine Komplikationen auftreten.

Es ist eine Aufgabe dieser Erfindung, ein Verfahren und eine Vorrichtungsanordnung zur Bestimmung der Hornhautdicke eines Auges vorzuschlagen, welche mindestens gewisse der oben beschriebenen Nachteile des Stands der Technik nicht aufweisen und welche insbesondere eine flächige Bestimmung der aktuellen Hornhautdicke eines menschlichen Auges mit einer hohen Ortsauflösung ermöglichen. Es ist eine weitere Aufgabe der vorliegenden Erfindung, den gerätetechnischen Aufwand für die Bestimmung der Hornhautdicke eines Auges zu reduzieren, um insbesondere die Realisierung von Handgeräten für die flächige Hornhautdickenmessung mit einer hohen Ortsauflösung zu ermöglichen.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Insbesondere werden diese Ziele durch die Erfindung dadurch erreicht, dass ein Kontaktkörper auf mindestens einen Kontaktbereich der Hornhaut aufgesetzt wird, und dass Lichtwellen von einer Lichtquelle durch den Kontaktkörper hindurch auf und in die Hornhaut gestrahlt werden. Lichtwellen, die von der Hornhaut und dem Kontaktkörper reflektiert werden, können dann durch Erfassungsmittel erfasst werden und auf Grund der durch den Kontaktkörper geschaffenen definierten Messbedingungen einer einfachen Bestimmung der Dicke der Hornhaut zugänglich gemacht werden. Es erübrigen sich beispielsweise die Bestimmung der Oberflächenneigung der Hornhaut oder die gebogene Ausführung von Projektionsschlitzen zur Erfüllung der Scheimpflugbedingung oder ein Autofokussystem für die Erfassungsmittel. Zudem ermöglicht der Kontaktkörper während der Messung unerwünschte Augenbewegungen und Verformungen des Auges, beispielsweise durch Augenfokussierung, leichter zu verhindern.

In einer bevorzugten Ausführungsvariante werden die Lichtwellen, die von der Lichtquelle ausgestrahlt werden, mindestens punktweise in einem zweidimensionalen Messbereich des Kontaktbereichs der Hornhaut, auf dem der Kontaktkörper aufgesetzt ist, auf und in die Hornhaut gestrahlt und die Hornhautdicke an einer oder mehreren Stellen des Messbereichs bestimmt. Dadurch kann die Hornhautdicke mit einer hohen Ortsauflösung bestimmt werden, beispielsweise flächendeckend innerhalb des Messbereichs.

In einer bevorzugten Ausführungsvariante wird ein Kontaktkörper verwendet, der eine vordefinierte Dicke aufweist, und die Dicke des Kontaktkörpers wird zur Bestimmung der Hornhautdicke mitverwendet. Bei bekannter lokaler Dicke des Kontaktkörpers unter Verwendung optischer Verfahren, die auf spiegelnder Reflexion an den Grenzflächen des Kontaktkörpers und der Hornhaut beruhen und bei denen die Dicke des Kontaktkörpers auf Grund von vom Kontaktkörper reflektierten Lichtwellen miterfasst werden kann, ist keine Kenntnis des Einfallswinkels von Lichtstrahlen an den Grenzflächen erforderlich und nur die Brechungsindizes des Kontaktkörpers und der Hornhaut müssen bekannt sein. Diese Ausführungsvariante weist somit den Vorteil auf, dass der in den bekannten Verfahren notwendige Aufwand zur Bestimmung der lokalen Oberflächenneigung der Hornhaut entfällt. Weist der Kontaktkörper zudem den gleichen Brechungsindex auf wie die Hornhaut, so muss nur das Verhältnis der Weglängen gemessen werden, die durch die in den Kontaktkörper und in die Hornhaut eingestrahlten Lichtwellen im Kontaktkörper, respektive in der Hornhaut, zurückgelegt werden, und auch Verfahren, die auf Lichtstreuung oder Fluoreszenz in der Hornhaut und im Kontaktkörper oder auf diffuser Reflexion an deren Grenzflächen beruhen, bedürfen dann keiner Kenntnis der lokalen Oberflächenneigung.

Zudem müssen auch die Abbildungsmassstäbe, das heisst das Verhältnis von Objektgrösse zu Bildgrösse, nicht bekannt sein, um die Hornhautdicke zu bestimmen. Zum Beispiel können so mit Hilfe einer Kontaktlinse bekannter Dicke und gleichem Brechungsindex wie die Hornhaut in einem herkömmlichen Spaltlampenbild Hornhautdickenmessungen durchgeführt werden.

In einer Ausführungsvariante wird die Hornhaut durch die Kontaktfläche des Kontaktkörpers in eine definierte Lage gebracht, das heisst die Form und/oder Position der Hornhaut wird durch die Kontaktfläche des Kontaktkörpers bestimmt. Auf Grund der definierten Lage, die je nach Ausführung a priori bekannt oder bestimmbar ist, lässt sich die lokale Oberflächenneigung der Hornhaut und der lokale Abbildungsmassstab ohne hohen gerätetechnischen Aufwand bestimmen. Bei optischen Verfahren, die auf spiegelnder Reflexion an Grenzschichten beruhen, lassen sich somit die von der Oberflächenneigung abhängigen Lauflängen der Lichtwellen in der Hornhaut bestimmen und daraus, in Kenntnis des Brechungsindexes der Hornhaut und des Kontaktkörpers, die Hornhautdicke (senkrecht zur Oberfläche der Hornhaut) bestimmen. Bei Verfahren mit Lichtstreuung oder Fluoreszenz in der Hornhaut oder diffuser Reflexion an den Grenzflächen ist neben dem Einfallswinkel der einfallenden Lichtstrahlen auch der Austrittswinkel (Brechungswinkel) der Lichtstrahlen aus dem Kontaktkörper, sowie die Kenntnis der lokalen Kontaktkörperdicke und der Brechungsindizes des Kontaktkörpers und der Hornhaut erforderlich. Zudem muss bei nicht telezentrischer Abbildung durch die Erfassungsmittel die Kontaktkörperdicke und der Abbildungsmassstab bekannt sein. Wird der Kontaktkörper geeignet ausgelegt, lassen sich eine Vielzahl dieser Parameter einfach und effizient festlegen.

In einer bevorzugten Ausführungsvariante wird ein planparalleler Kontaktkörper verwendet, dessen Dicke bei der Messung miterfasst wird. Die Verwendung eines planparallelen Kontaktkörpers erweist sich insbesondere auch für Verfahren mit Lichtstreuung oder Fluoreszenz oder diffuser Reflexion als besonders vorteilhaft, da die oben angeführten erforderlichen Messgrössen, respektive Messparameter, mit einem bedeutend geringeren gerätetechnischen Aufwand als in bekannten Verfahren erhalten werden können. Die Verwendung eines planparallelen Kontaktkörpers zum Aufsetzen auf die Hornhaut ermöglicht Ausführungsvarianten, in denen der Beleuchtungswinkel (Einfallswinkel) und der Beobachtungswinkel und/oder der Objektabstand konstant gehalten werden können (bei zum Kontaktkörper paralleler Verschiebung der optischen Messvorrichtung, die die Lichtquelle und die Erfassungsmittel enthält), so dass eine aufwendige Kalibrierung der Messvorrichtungsanordnung entfällt. Ausserdem muss der Patient nicht aufwendig zu der Messvorrichtung ausgerichtet werden.

In einer Ausführungsvariante wird der Kontaktkörper auf der Hornhaut oder dem Auge fixiert, beispielsweise mittels Unterdruck (Saugring). Dies hat den Vorteil, dass ein fest definierter Zustand der Hornhaut erreicht wird und dass das Messverfahren langsamer ausgeführt werden kann, da keine Relativbewegungen zwischen dem Auge und dem Kontaktkörper während der Messung auftreten. Zudem wird durch die Fixierung des Kontaktkörpers auf der Hornhaut oder dem Auge verhindert, dass Augendruckschwankungen, beispielsweise durch den Puls verursacht, und Fokussierung des Auges die Form und die Position der Hornhaut verändern.

In einer Ausführungsvariante wird ein semitransparenter Kontaktkörper verwendet. Ein semitransparenter Kontaktkörper bewirkt, dass einfallendes Licht gestreut wird, so dass optische Verfahren basierend auf Lichtstreuung oder Fluoreszenz oder diffuser Reflexion angewandt werden können.

In einer Ausführungsvariante wird ein Kontaktkörper verwendet, dessen Brechungsindex dem Brechungsindex der Hornhaut entspricht. Diese Ausführungsvariante hat den Vorteil, dass die Hornhautdicke sowohl unabhängig vom Einfallswinkel, respektive Reflexionswinkel der Lichtstrahlen als auch unabhängig von den Brechungsindizes für den Kontaktkörper und die Hornhaut bestimmt werden kann.

In einer Ausführungsvariante werden die Lichtquelle und/oder die Erfassungsmittel gemäss der Scheimpflugbedingung angeordnet, um Defokussierungen zu vermeiden. Durch die Scheimpfluganordnung lassen sich bei zueinander geneigter Objekt- und Bildebene schärfere Abbildungen erzeugen, als dies mit anderen Konfigurationen, beispielsweise einer senkrechten Anordnung der Bildebene zur optischen Achse des abbildenden Systems, möglich ist.

In einer Ausführungsvariante werden die Lichtwellen, die von der Lichtquelle ausgestrahlt werden, mit konstantem Einfallswinkel auf die Hornhaut gestrahlt. Dadurch kann die Bestimmung der Hornhautdicke vereinfacht werden, da der Einfallswinkel nur einmalig bestimmt werden muss.

In einer Ausführungsvariante werden die reflektierten Lichtwellen unter konstantem Beobachtungswinkel erfasst. Dadurch kann die Bestimmung der Hornhautdicke vereinfacht werden, da der Beobachtungswinkel nur einmalig bestimmt werden muss.

In einer Ausführungsvariante wird die Lichtquelle äquidistant zur Kontaktfläche des Kontaktkörpers verschoben. Der Vorteil dieser Ausführungsvariante besteht darin, dass eine höhere Auflösung, respektive eine grössere Messgenauigkeit erreicht werden kann, da es keine vertikalen Relativbewegungen zwischen der Hornhaut und der Lichtquelle gibt und Lichtwellen sehr fein fokussiert werden können.

In einer Ausführungsvariante werden die Erfassungsmittel äquidistant zur Kontaktfläche des Kontaktkörpers verschoben. Der Vorteil dieser Ausführungsvariante besteht darin, dass eine höhere Auflösung, respektive eine grössere Messgenauigkeit erreicht werden kann, da es keine vertikalen Relativbewegungen zwischen der Hornhaut und den Erfassungsmitteln gibt und der Tiefenschärfebereich der Erfassungsmittel klein und damit die optische Auflösung hoch ausgelegt werden kann.

In einer Ausführungsvariante ist die optische Messvorrichtung, die die Lichtquelle und die Erfassungsmittel umfasst, mit dem Kontaktkörper verbunden, wobei die Lichtquelle und/oder die Erfassungsmittel relativ zum Kontaktkörper beweglich sind. Wenn diese Verbindung nur während der Lagebestimmung des Kontaktkörpers und der anschliessenden Messung fest ist, können demontierbare Kontaktkörper verwendet werden, die beispielsweise verschiedene vordefinierte Dicken aufweisen und/oder wegwerfbar sind.

Nachfolgend wird eine Ausführung der vorliegenden Erfindung anhand eines Beispieles beschrieben. Das Beispiel der Ausführung wird durch folgende beigelegten Figuren illustriert:

Figur 1 zeigt ein Blockdiagramm, das schematisch die Vorrichtungsanordnung zur Bestimmung der Hornhautdicke eines Auges illustriert, welche Vorrichtungsanordnung eine optische Messvorrichtung mit einer Lichtquelle und mit Erfassungsmitteln sowie einen Kontaktkörper umfasst.

Figur 2 zeigt ein Blockdiagramm, das schematisch eine Ausführungsvariante der Vorrichtungsanordnung illustriert, in der die optische Messvorrichtung mittels eines Körpers fest mit dem Kontaktkörper verbunden ist.

Figur 3 zeigt ein Blockdiagramm, das schematisch einen Kontaktkörper illustriert, der durch eine Vorrichtung auf dem Auge, respektive auf der Hornhaut fixiert ist.

Figur 4 zeigt ein Blockdiagramm, das schematisch eine optische Messvorrichtung illustriert, die einen optischen Messkopf mit einer Lichtquelle und mit Erfassungsmitteln umfasst und die einen Messbereich punktweise erfasst.

Figur 5 zeigt ein Blockdiagramm, das schematisch einen zweidimensionalen Messbereich illustriert, in dem entlang einer Linie gemessen wird.

Figur 6 zeigt ein Schichtenmodell, das schematisch die Verwendung des Kontaktkörpers als Kalibrierkörper bei der Bestimmung der Hornhautdicke basierend auf spiegelnder Reflexion an den Grenzschichten illustriert.

Figur 7 zeigt ein Schichtenmodell, das schematisch die Verwendung des Kontaktkörpers als Kalibrierkörper bei der Bestimmung der Hornhautdicke basierend auf Lichtstreuung, Fluoreszenz oder diffuser Reflexion illustriert.

Figur 8a zeigt eine Seitenansicht, die schematisch eine Ausführungsvariante illustriert, in der der Kontaktkörper einen semitransparenten Bereich aufweist, der ausserhalb des Messbereichs der Hornhaut liegt, auf dem der Kontaktkörper auf die Hornhaut aufgesetzt ist.

Figur 8b zeigt eine Draufsicht, die schematisch eine Ausführungsvariante illustriert, in der der Kontaktkörper einen semitransparenten Bereich aufweist, der ausserhalb des Kontaktbereichs der Hornhaut liegt, auf dem der Kontaktkörper auf die Hornhaut aufgesetzt ist.

Figur 9a zeigt eine Draufsicht, die schematisch einen Kontaktkörper mit Referenzmarken illustriert.

Figur 9b zeigt eine Seitenansicht, die schematisch einen Kontaktkörper mit Referenzmarken illustriert, der auf einer Hornhaut aufgesetzt ist.

Figur 10 zeigt ein Diagramm, das schematisch eine Vorrichtungsanordnung gemäss der Scheimpflugbedingung illustriert.

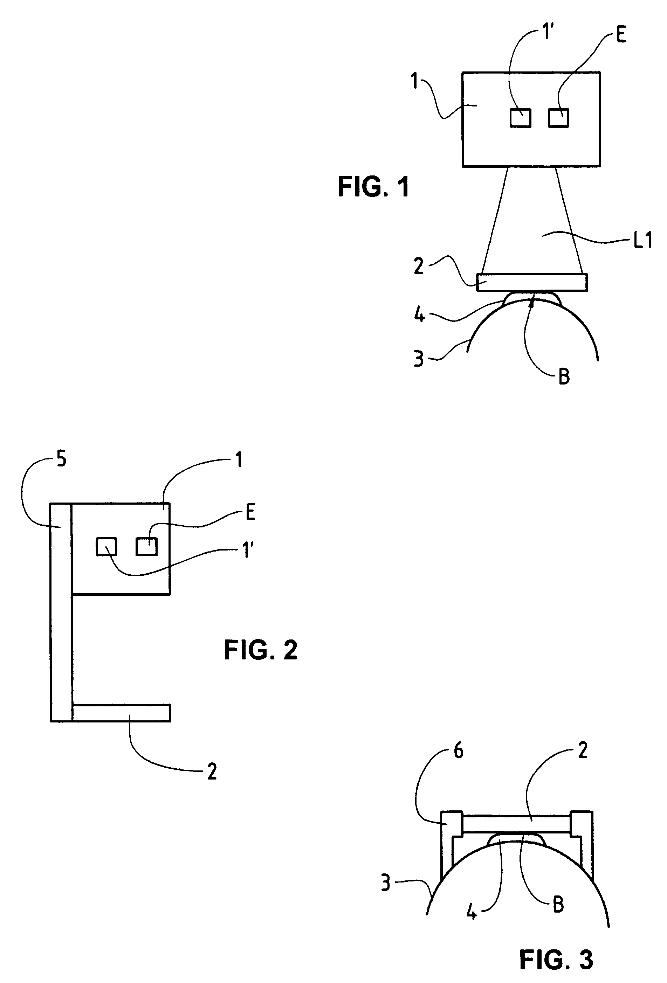

In der Figur 1 bezieht sich das Bezugszeichen 1 auf eine optische Messvorrichtung 1 mit einer Lichtquelle 1', die Lichtwellen L1 auf und in die Hornhaut 4 des Auges 3 strahlt. Die Lichtquelle 1' ist nur schematisch dargestellt und umfasst neben Mitteln zur Erzeugung der Lichtwellen L1 auch die zugehörigen Abbildungsmittel, beispielsweise eine oder mehrere Linsen. Das Auge 3 ist insbesondere ein menschliches Auge. Wie in der Figur 1 dargestellt ist, ist auf der Hornhaut 4 ein Kontaktkörper 2 auf einen Kontaktbereich B der Hornhaut 4 aufgesetzt. Der Kontaktkörper 2 ist vorzugsweise eine planparallele Platte aus optisch transparentem Material, beispielsweise Glas oder Kunststoff, die die Hornhaut 4 im Kontaktbereich B in einen applanierten Zustand versetzt. Es sind auch ein- oder beidseitig gekrümmte Kontaktkörper 2 möglich, beispielsweise in der Form einer konkaven Linse. Ein linsenförmiger Kontaktkörper 2 kann beispielsweise so ausgestaltet sein, dass er die Lichtwellen L1 so ablenkt, dass sie senkrecht zur Hornhaut 4 auftreffen. Der Kontaktkörper 2 könnte auch so ausgeführt sein, dass er die Hornhaut 4 in mehreren Kontaktbereichen B kontaktiert. Die Lichtwellen L1 werden von der Lichtquelle 1' durch den Kontaktkörper 2 hindurch auf und in die Hornhaut 4 gestrahlt. Die optische Messvorrichtung 1 umfasst zudem schematisch dargestellte Erfassungsmittel E zur Erfassung von Lichtwellen, die von der Hornhaut 4 und vom Kontaktkörper 2 reflektiert werden. Die Erfassungsmittel E hängen vom verwendeten optischen Verfahren ab. Die Erfassungsmittel E umfassen beispielsweise eine CCD-Kamera und sind beispielsweise zu Steuerungs- und Auswertungszwecken mit entsprechenden Verarbeitungsmitteln verbunden.

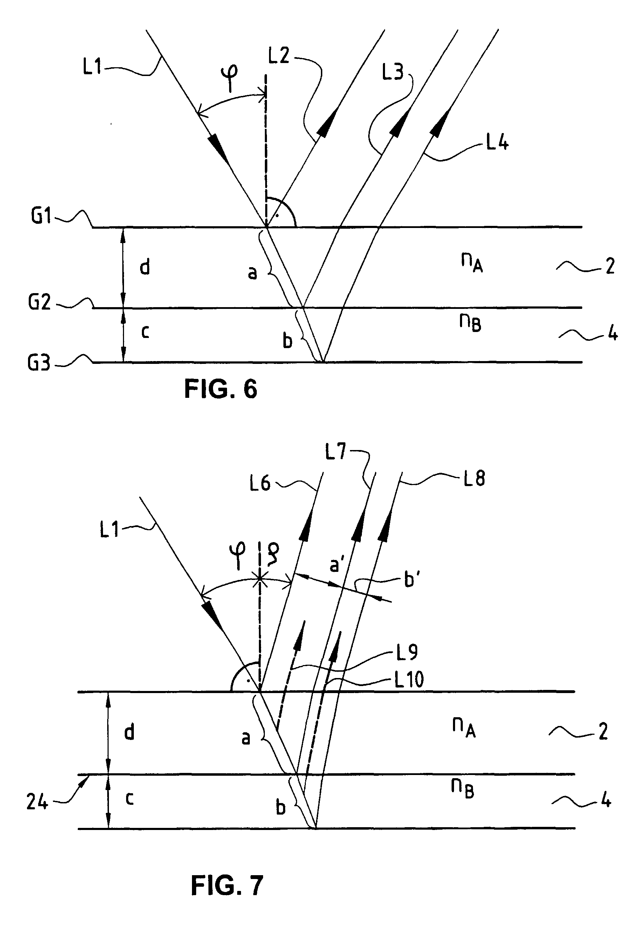

In der Figur 6 wird schematisch die Verwendung des Kontaktkörpers 2 als Kalibrierkörper bei der Bestimmung der Dicke c der Hornhaut 4 basierend auf spiegelnder Reflexion an den Grenzflächen G1, G2 und G3 illustriert. Der Kontaktkörper 2 weist eine vordefinierte Dicke d (entweder lokal, das heisst die Dicke d des Kontaktkörpers 2 ist zumindest an den Messpunkten bekannt, aber nicht überall gleich, oder global, das heisst der Kontaktkörper weist eine konstante Dicke d auf) und einen Brechungsindex $n_A$ auf. Der Brechungsindex der Hornhaut 4 ist $n_B$. Ein von der Lichtquelle 1' abgestrahlter Lichtstrahl L1, der beispielhaft für alle von der Lichtquelle 1' abgestrahlten (ersten) Lichtwellen steht, trifft mit einem Einfallswinkel $\varphi$ auf den Kontaktkörper 2 und wird dort an der Grenzfläche G1 teilweise als Lichtstrahl L2 (dritte Lichtwellen) reflektiert. Der in den Kontaktkörper 2 und in die Hornhaut 4 einfallende Teil des Lichtstrahls L1 legt dort die zusätzlichen Weglängen a, respektive b, zurück und wird durch die Hornhaut 4 an den Grenzflächen G2 und G3 teilweise reflektiert. In der Figur 6 sind die von der Hornhaut 4 reflektierten und aus dem Kontaktkörper 2 austretenden Lichtstrahlen mit L3, respektive L4, bezeichnet (zweite Lichtwellen). Der Tränenfilm zwischen dem Kontaktkörper 2 und der Hornhaut 4 ist in der Figur 6 nicht dargestellt (falls die Dicke des Tränenfilms berücksichtigt würde, gäbe es sowohl eine Reflexion an der Grenzschicht zwischen dem Kontaktkörper und der Tränenflüssigkeit, als auch an der Grenzschicht zwischen der Tränenflüssigkeit und der Hornhaut). Es sei angemerkt, dass die Lichtstrahlen L1 der Lichtquelle 1' im allgemeinen nicht parallel zueinander sind. Die Weglängen a und b können beispielsweise durch Autofokusverfahren (beispielsweise in CD-Spielern verwendet), durch interferometrische Verfahren mit geringer Kohärenzlänge (optical coherence tomography, OCT; Weisslichtinterferometrie z.B. mittels eines Fabry-Pérot Fiber Optic Sensors vertrieben durch die Firma Fiso Technologies Inc.), durch interferometrische Mehrwellenlängenverfahren, durch konfokale Verfahren mit wellenlängenabhängigem Fokus (z.B. basierend auf Weisslichtquelle und Spektrometer) oder Verfahren mit einfachem Fokus (Messung des Amplitudenmaximums, beispielsweise mittels Konfokalmikroskop), durch Laufzeitverfahren (z. B. mit Femtopulsen) oder durch Reflexionsverfahren (z. B. nach dem Prinzip des Laser-Reflex-Sensors der Firma Schäfter+Kirchhoff GmbH, Celsiusweg 15, D22761 Hamburg) unter Verwendung der entsprechenden Erfassungsmittel E und Verarbeitungsmittel gemessen werden. Die Dicke c der Hornhaut 4 ergibt sich aus den Weglängen a und b, aus der Dicke d des Kontaktkörpers, sowie aus den Brechungsindizes $n_A$ und $n_B$ des Kontaktkörpers 2, respektive der Hornhaut 4:

$$c = b\sqrt{1 - \frac{n_A^2}{n_B^2} + \frac{n_A^2}{n_B^2}\frac{d^2}{a^2}}$$

Die oben stehende Beziehung ist unabhängig vom Einfallswinkel φ, respektive vom gleich grossen Reflexionswinkel. Bei grossem oder sich änderndem Einfallswinkel φ, respektive Reflexionswinkel, muss allerdings darauf geachtet werden, dass die für das gewählte Verfahren verwendeten Erfassungsmittel E alle reflektierten Strahlen L2, L3, L4 erfassen können. Abhängig von den eingesetzten Verfahren treten Abweichungen bei der Messung von a und b auf. Insbesondere können bei nicht senkrechtem Einfall der Lichtwellen L1 Abweichungen auftreten. Unter Kenntnis des Einfallswinkels φ lassen sich diese Abweichungen korrigieren. Der Winkel φ lässt sich beispielsweise bei interferometrischen Verfahren oder Laufzeitverfahren mittels der bekannten Kontaktkörperdicke d bestimmen. Leitet man die Gleichungen für die Laufzeitunterschiede der in der Figur 6 dargestellten Lichtstrahlen L2 und L3 her, so ergibt sich mit Hilfe des Brechungsgesetzes bei bekanntem Brechungsindex $n_A$ und bekannter Dicke d des Kontaktkörpers 2 eine Beziehung zur Bestimmung des Einfallswinkels φ. Durch die Verwendung eines Kontaktkörpers 2 mit bekannter Dicke d als Referenz, ergibt sich also der Vorteil, dass die Dicke c der Hornhaut 4 unabhängig von der Oberflächenneigung gemessen werden kann.

Unter der Voraussetzung, dass der nicht dargestellte Tränenfilm zwischen dem Kontaktkörper 2 und der Hornhaut 4 eine ausreichende Reflexion ergibt oder ein reflexverstärkendes Produkt dem Tränenfilm zugegeben wird oder der Kontaktkörper 2 entsprechend beschichtet wird, kann das Verfahren weiter vereinfacht werden, indem ein Kontaktkörper 2 mit einem Brechungsindex $n_A$ gewählt wird, der dem Brechungsindex $n_B$ der Hornhaut 4 entspricht. Dann ergibt sich die Dicke c der Hornhaut 4 aus den Weglängen a und b, sowie aus der Dicke d des Kontaktkörpers 2:

$$c = b\frac{d}{a}$$

Es ist ersichtlich, dass nicht die tatsächlichen Längen a und b für die Messung entscheidend sind, sondern nur deren Verhältnis. Damit spielen multiplikative Abweichungen bei der Messung von a und b, wie sie zum Beispiel durch die Änderung des Einfallswinkels φ auftreten können, keine Rolle mehr.

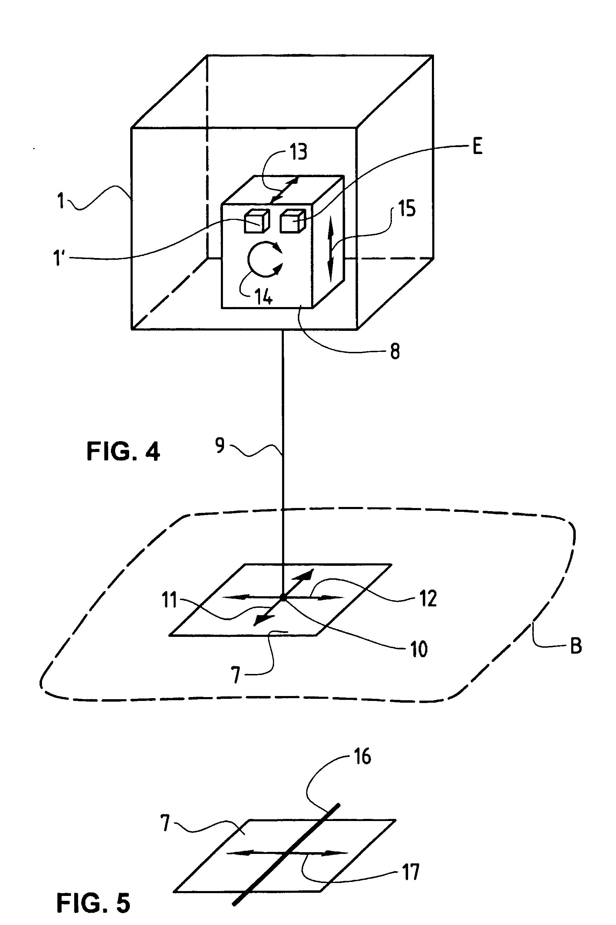

Sind der Reflexionswinkel, der Brechungsindex $n_B$ der Hornhaut 4 und der Brechungsindex $n_A$ des Kontaktkörpers 2 bekannt, dann lässt sich die Dicke c der Hornhaut 4 auch nur aus der der Weglänge b entsprechenden Messgrösse bestimmen. Im Falle eines planparallelen Kontaktkörpers 2 kann der Reflexionswinkel beispielsweise durch eine parallele Verschiebung (translatorisch) des für das optische Messverfahren verwendeten Messkopfs 8 (siehe Figur 4), respektive der Lichtquelle 1', konstant gehalten werden. Ist das Messverfahren darüber hinaus auch noch vom Arbeitsabstand abhängig, wie zum Beispiel konfokale Verfahren, dann muss die Verschiebung zusätzlich äquidistant ausgeführt werden. Lässt sich weiterhin die Position des Kontaktkörpers 2 bestimmen und ist die Kontaktfläche G2 hinreichend genau bekannt, so kann diese als Referenz verwendet werden, bezüglich derer die Fläche G3 der Hornhaut 4 vermessen werden kann. Dies ist besonders dann von Vorteil, wenn sich die Lagebeziehung zwischen dem für das optische Messverfahren verwendeten Messkopf 8 (siehe Figur 4) und dem Kontaktkörper 2 nicht ändert. Beispielsweise müsste ein Autofokusverfahren so nur vor der Vermessung der Hornhaut 4 die Position des Kontaktkörpers 2 einmal bestimmen und könnte dann ausschliesslich die Fläche G3 vermessen.

In der Figur 7 wird schematisch die Verwendung des Kontaktkörpers 2 als Kalibrierkörper bei der Bestimmung der Dicke c der Hornhaut 4 basierend auf Lichtstreuung, Fluoreszenz oder diffuser Reflexion illustriert. Lichtstreuung und Fluoreszenz können in der Hornhaut 4 und im Kontaktkörper 2 auftreten. Um eine ausreichende Streuung des einfallenden Lichtstrahls L1, der beispielhaft für alle von der Lichtquelle 1' abgestrahlten (ersten) Lichtwellen steht, zu erreichen, wird vorzugsweise ein semitransparenter Kontaktkörper 2 verwendet. Es können aber auch fluoreszierende oder kontrastverstärkende Mittel in den Tränenfilm gegeben werden. Der Tränenfilm zwischen dem Kontaktkörper 2 und der Hornhaut 4 ist in der Figur 7 nicht dargestellt. Diffuse Reflexion kann an den Grenzflächen des Kontaktkörpers 2 erzeugt werden. Gemessen werden die in der Figur 7 dargestellten Distanzen a' und b' zwischen den reflektierten Lichtstrahlen L6 (dritte Lichtwellen), L7 und L8 (zweite Lichtwellen), beispielsweise über eine CCD-Kamera. Die in der Figur 7 gestrichelt dargestellten Lichtstrahlen (Lichtwellen) L9 und L10 sollen andeuten, dass bei Verfahren, die auf Lichtstreuung oder Fluoreszenz basieren, Lichtwellen auch im Innern des Kontaktkörpers 2, respektive im Innern der Hornhaut 4, gestreut werden. Es sei angemerkt, dass die Strahlen L6, L7, L8 nur die Hauptstrahlen des optischen Systems der Erfassungsmittel E darstellen, wobei die Gesamtheit der reflektierten, respektive gestreuten Lichtstrahlen L6, L7, L8 in einen weiteren Raumbereich gestrahlt werden. Abhängig vom optischen System sind die Hauptstrahlen L6, L7, L8 wie in der Figur 7 dargestellt parallel (telezentrische Abbildung) oder konvergent. Bei konvergenten Strahlen sind a' und b' nicht mehr konstant und es wird das vom Abbildungsmassstab abhängige Abbild der Strecken a und b gemessen. Auch bei dieser Art Verfahren ergibt sich eine vereinfachte Auswertung, wenn ein Kontaktkörper 2 mit einem Brechungsindex $n_A$ gewählt wird, der dem Brechungsindex $n_B$ der Hornhaut 4 entspricht. Dann ergibt sich die Dicke c der Hornhaut 4 aus den gemessenen Weglängen a' und b', sowie aus der Dicke d des Kontaktkörpers 2:

$$c = b' \frac{d}{a'}$$

Wie bei Verfahren mit spiegelnder Reflexion ergibt sich der Vorteil, dass nur das Verhältnis der Strecken a' und b', respektive der Abbilder von a und b, gemessen werden muss. Bei Systemen mit nicht telezentrischer Abbildung muss der aktuelle Abbildungsmassstab bei der alleinigen Messung von a und b bekannt sein, nicht aber wenn nur das Verhältnis bestimmt wird. Wenn a und b bei der Abbildung dicht nebeneinander liegen, spielen auch verzerrte Abbildungen keine Rolle, da in kleinen Bildbereichen lineare Zusammenhänge angenommen werden können und somit das Verhältnis von a und b genau bestimmt werden kann.

Sind $n_A$ und $n_B$ nicht gleich, müssen zusätzlich zu den Brechungsindizes $n_A$ und $n_B$ und der Dicke d des Kontaktkörpers 2 der Einfallswinkel φ und der Austrittswinkel ρ (Brechungswinkel) bekannt sein. Eine präzise Messung der Winkel φ und ρ lässt sich beispielsweise über den Kontaktkörper 2 ausführen, da dessen Oberfläche im Vergleich zu der Oberflächengestalt der Hornhaut 4 sehr präzise gefertigt werden kann. Werden die Winkel φ und ρ konstant gehalten, beispielsweise durch eine äquidistante (parallele) Verschiebung des für das optische Messverfahren verwendeten Messkopfs 8 (siehe Figur 4), respektive der Lichtquelle 1', zur Kontaktfläche des (planparallelen) Kontaktkörpers 2, dann lässt sich die Dicke c der Hornhaut 4 nach einmaliger Kalibrierung (respektive Bestimmung von φ und ρ) auch nur aus der zusätzlichen Weglänge b' bestimmen. Für die Messung der Distanz b' ergeben sich günstigere Bedingungen, wenn $n_B > n_A$ gewählt wird (der Wert von b' wird grösser). Bei nicht telezentrischer Abbildung muss wiederum der Abbildungsmassstab bekannt sein. Wiederum ergeben sich die oben genannten Vorteile, wenn sich die Position des Kontaktkörpers 2 bestimmen lässt und die Kontaktfläche 24 hinreichend genau bekannt ist. Somit kann auch hier die Kontaktfläche 24 als Referenz verwendet werden, bezüglich derer die Hornhaut 4 vermessen werden kann. Es sei hier zudem noch erwähnt, dass die Winkel φ und ρ anhand trigonometrischer Beziehungen bestimmt werden können. Betrachtet man beispielsweise in der Figur 7 das von den Lichtstrahlen L1, L6 und L7 im Kontaktkörper 2 beschriebene Dreieck, so lässt sich bei gegebener Weglänge a', Dicke d und Brechungsindex $n_A$ einer der Winkel φ und ρ über trigonometrische Beziehungen als Funktion des anderen berechnen, das heisst nur einer der beiden Winkel φ und ρ muss bekannt sein. Es ergeben sich also auch hier Vorteile aus der Verwendung von Kontaktkörpern 2 mit bekannter Dicke d.

Die folgenden optischen Verfahren, die auf diffuser Reflexion respektive Lichtstreuung beruhen, können beispielsweise verwendet werden: ein einzelner Lichtstrahl (in Kombination mit einem eindimensionalen Scanner als Lichtschnitt, in Kombination mit einem zweidimensionalen Scanner zur Flächenerfassung), mehrere Lichtstrahlen, Lichtschnittverfahren (z.B. basierend auf Spaltlampe oder eindimensional gescanntem einzelnem Lichtstrahl; in Kombination mit einem eindimensionalen Scanner zur Flächenerfassung), Streifenprojektionsverfahren (mehrere Lichtschnitte) oder photogrammetrische Verfahren mit strukturierter Beleuchtung.

Die folgenden optischen Verfahren, die auf Fluoreszenz beruhen, können beispielsweise verwendet werden: Multiphotonenanregung über fokussiertes Licht oder Multiphotonenanregung nur im Fokus bei Verwendung von kurzen Pulsen (Femtopulse).

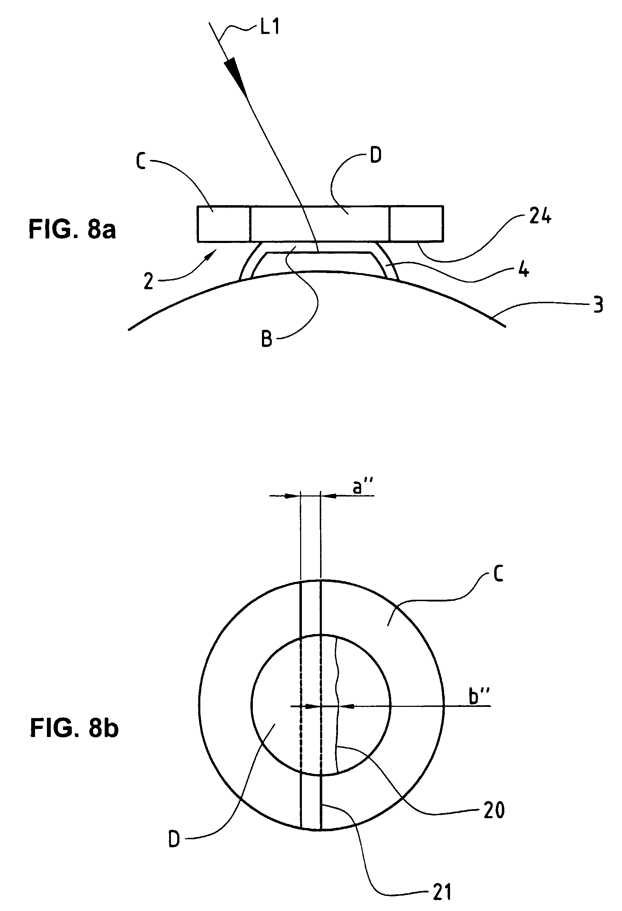

In den Figuren 8a und 8b wird schematisch eine Ausführungsvariante für Verfahren basierend auf Lichtstreuung oder Fluoreszenz oder diffuser Reflexion illustriert, in der der Kontaktkörper 2 einen semitransparanten Bereich C aufweist, der einen transparenten Bereich D des Kontaktkörpers 2 über dem Kontaktbereich B (beispielsweise der applanierte Bereich B in der Figur 8a) der Hornhaut 4 umgibt. Gemessen werden die in der Figur 8b dargestellten Distanzen a" und b", wobei sich die Distanz b" lokal in Abhängigkeit von der Hornhautdicke ändert. Diese Ausführungsvariante hat den Vorteil, dass auch bei der Verwendung eines breiten Lichtstrahls die Linie 21 im Bereich C sowie die Linie 20 im Bereich D einen sehr guten Kontrast zum Hintergrund aufweisen und somit gut detektiert werden können. Vorteil dieser Variante ist, dass der semitransparente Bereich C des Kontaktkörpers 2 nicht den zu vermessenden Bereich B der Hornhaut 4 verdeckt.

Wie in der Figur 4 dargestellt wird umfasst die optische Messvorrichtung 1 einen optischen Messkopf 8 mit der Lichtquelle 1'. Der optische Messkopf 8, respektive die Lichtquelle 1', ist so eingerichtet, dass die Lichtwellen L1 mindestens punktweise in einem zweidimensionalen Messbereich 7 des Kontaktbereichs B, in und auf die Hornhaut 4 gestrahlt werden können, so dass die Dicke der Hornhaut 4 an einer oder mehreren Stellen des Messbereichs 7 bestimmt werden kann. Bei einer Ausführung mit mehreren Kontaktbereichen B könnte der Messbereich auch Bereiche zwischen den Kontaktbereichen umfassen. Je nach Ausführungsvariante, beispielsweise je nachdem ob ein punkt-, linien- (streifen-) oder flächenförmig arbeitendes optisches Verfahren eingesetzt wird, wird der optische Messkopf 8 zur Erfassung des Messbereichs 7 mechanisch bewegt. Der optische Messkopf 8 umfasst zudem die vom verwendeten optischen Verfahren abhängigen Erfassungsmittel E zur Erfassung der Lichtwellen L3 und L4 (siehe Figur 6), respektive L7, L8 und L10 (siehe Figur 7), die von der Hornhaut 4 reflektiert werden, und der Lichtwellen L2 (siehe Figur 6), respektive L6 und L9 (siehe Figur 7), die vom Kontaktkörper 2 reflektiert werden. In einer Ausführungsvariante bewegen sich Lichtquelle 1' und Erfassungsmittel E mit dem optischen Messkopf 8 mit. In einer weiteren Ausführungsvariante wird nur die Lichtquelle 1' bewegt und die Erfassungsmittel E bleiben ortsfest. In der Figur 4 wird ein punktförmig arbeitendes Verfahren mit einem von der Lichtquelle ausgestrahlten Lichtstrahl 9, dem Messpunkt 10 und den Verfahrrichtungen 11 und 12 im Messbereich 7 der Hornhaut 4 dargestellt. Wie in der Figur 4 beispielhaft angedeutet wird, führt der optische Messkopf 8 für die Richtung 11 eine Translation 13 und führt der optische Messkopf 8, respektive die Lichtquelle 1', für die Richtung 12 eine Rotation 14 durch. Die Bewegungen für die Richtungen 11 und 12 lassen sich auch durch zwei Translationen oder durch zwei Rotationen ausführen. Für bestimmte Anwendungen, beispielsweise bei einer Schiefstellung des Kontaktkörpers 2, kann zusätzlich auch eine vertikale Verfahrbewegung 15 erforderlich sein, um den Messpunkt im vertikalen Messbereich zu halten.

In der Figur 5 wird ein linienförmig (streifenförmig) arbeitendes Verfahren dargestellt, zum Beispiel mit einem von der Lichtquelle 1' ausgestrahlten Lichtschnitt 16, bei dem nur die Bewegungsrichtung 17 zur Erfassung des Messbereichs 7 verbleibt, wenn die Länge des Lichtschnitts 16 die Breite des Messbereichs 7 abdeckt. Die verbleibende Bewegungsrichtung 17 kann durch entsprechende Bewegung des optischen Messkopfs 8, respektive der Lichtquelle 1' ausgeführt werden. Bei einem nicht dargestellten flächenförmig arbeitenden Verfahren, bei dem Bereiche, die grösser als der Messbereich 7 sind, ausgeleuchtet werden, kann ganz auf Verfahrbewegungen verzichtet werden.

Wie in der Figur 2 dargestellt wird, ist die optische Messvorrichtung 1 in einer Ausführungsvariante fest mittels eines Körpers 5 mit dem Kontaktkörper 2 verbunden. Für die oben vorgestellten Verfahren, die die Kenntnis der Lage des Kontaktkörpers 2 erfordern, braucht die Verbindung des Kontaktkörpers 2 mit der optischen Messvorrichtung 1 nur während der Lagebestimmung und der anschliessenden Messung fest zu sein. Das heisst eine Lagekalibrierung oder Langzeitstabilität ist nicht erforderlich, so dass demontierbare Kontaktkörper 2 verwendet werden können, die beispielsweise verschiedene vordefinierte Dicken d aufweisen und/oder wegwerfbar sind.

An dieser Stelle soll darauf hingewiesen werden, dass es auch möglich ist, die Lichtquelle 1' in den Kontaktkörper 2 zu integrieren, beispielsweise in der Ausführung einer oder mehrerer Leuchtdioden. In gleicher Weise lassen sich Empfangsmittel E in den Kontaktkörper 2 integrieren. Neuste Entwicklungen im Bereich der MEMS (Micro Electromechanical Systems) zeigen die Machbarkeit solcher Mikrointegrationen.

In der in der Figur 3 schematisch dargestellten Ausführungsvariante wird der Kontaktkörper 2 auf dem Auge 3, respektive auf der Hornhaut 4 durch Unterdruck fixiert, beispielsweise mittels eines dem Fachmann bekannten Saugrings 6. Wird der Kontaktkörper 2 sowohl auf der Hornhaut 4 fixiert als auch mit der optischen Messvorrichtung 1 verbunden, entfällt ein aufwendiges Tracking des Auges 3 während der Messung vollständig. Der Patient muss nicht aufwendig vor der Messvorrichtung positioniert werden, er kann selbst in einer liegenden Position untersucht werden und braucht zudem sein Auge 3 nicht auf ein Zielobjekt zu fokussieren.

Die optische Messvorrichtung 1 umfasst auch Verarbeitungsmittel, die vom verwendeten optischen Verfahren sowie von den dafür eingesetzten Erfassungsmitteln E abhängen. Die Verarbeitungsmittel umfassen beispielsweise einen Prozessor mit verbundenem Daten- und Programmspeicher, in dem ausführbare Softwareprogramme gespeichert sind. Die Softwareprogramme sind zur Steuerung des optischen Messkopfs 8, respektive der Lichtquelle 1', sowie zur Steuerung der Erfassungsmittel E und zur Auswertung der erfassten reflektierten Lichtwellen L2, L6 und L9 (dritte Lichtwellen) sowie L3, L4, L7, L8 und L10 (zweite Lichtwellen) für die Bestimmung der Hornhautdicke über einen zweidimensionalen Messbereich 7 (siehe Figur 4) eingerichtet. Die gemessenen Hornhautdicken können beispielsweise numerisch und/oder grafisch auf einem Bildschirm dargestellt oder ausgedruckt werden. In einer Ausführungsvariante können die Messdaten auch in Bezug zum betreffenden Auge 3 dargestellt werden, wobei sich als Referenzmarken des Auges 3 Strukturen wie die Iris, die Retina oder der Limbus eignen. Falls ein optisches Verfahren zur Bestimmung der Hornhautdicke verwendet wird, das nicht geeignet ist, solche Referenzmarken auf dem Auge 3 und auf dem Kontaktkörper 2 zu erzeugen oder zu erfassen, kann dies beispielsweise mittels einer zusätzlichen Kamera und einem geeignet angeordneten halbdurchlässigen Spiegel erreicht werden.

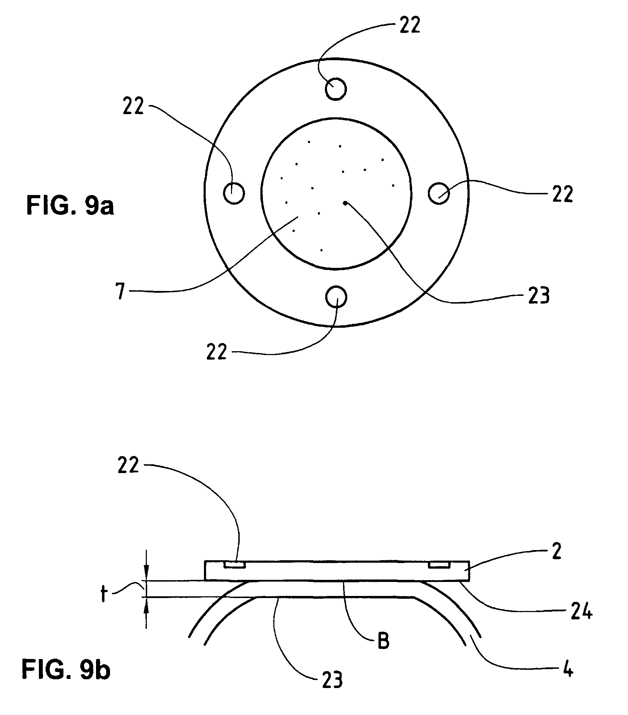

Referenzmarken, die zur Lagebestimmung des Kontaktkörpers 2 herangezogen werden, können auf dem Kontaktkörper 2 im Bereich über dem Kontaktbereich B (beispielsweise der applanierte Bereich B in der Figur 9b) der Hornhaut 4 oder ausserhalb dieses Bereichs angebracht werden. Referenzmarken können aktiv (das heisst selbstleuchtend oder aufprojiziert) oder passiv (das heisst indirekt beleuchtet) sein. Spiegelnde Reflexion, diffuse Reflexion, Lichtstreuung oder Fluoreszenz kann ausgenutzt werden um Referenzmarken zu erzeugen. In der Figur 9a ist beispielhaft ein Kontaktkörper 2 dargestellt, auf dem Referenzmarken 22 ausserhalb des Messbereichs 7 angebracht sind, wobei der Messbereich 7 ein Teilbereich des Kontaktbereichs B der Hornhaut 4 ist oder mit dem Kontaktbereich B übereinstimmt. Die Referenzmarken 22 auf dem Kontaktkörper 2 können dazu verwendet werden, die Lage des Kontaktkörpers 2 zu bestimmen und in Übereinstimmung mit der bestimmten Lage Messungen relativ zu der in der Figur 9b dargestellten Kontaktfläche 24, in diesem Beispiel eine ebene Applanationsfläche, durchzuführen. Daraus ergibt sich der Vorteil, dass nacheinanderfolgende Messungen, beispielsweise Messungen einzelner Messpunkte 23, bezüglich einer physikalisch existierenden Bezugsbasis, insbesondere der Kontaktfläche 24, erfasst werden können, so dass beispielsweise nur Tiefenmessungen t von Messpunkten 23 auf der inneren Hornhautoberfläche durchgeführt werden müssen, um die Hornhautdicke zu bestimmen. Da sich Referenzflächen mit sehr hoher Genauigkeit herstellen und bestimmen lassen, reduziert sich die Bestimmung der Hornhautdicke für einen Messpunkt 23 auf einen Messwert t, so dass eine höhere Genauigkeit erzielt werden kann als bei der Differenzbildung von zwei Messwerten für einen Messpunkt. Zudem kann der Messbereich des verwendeten optischen Verfahrens beschränkt werden, wenn nur die innere Hornhautoberfläche (Kontaktfläche 24) vermessen wird, beispielsweise müsste ein Autofokussensor oder Lichtschnittsensor nur auf die innere Hornhautoberfläche fokussieren, was zusätzlich die Messgeschwindigkeit erhöht.

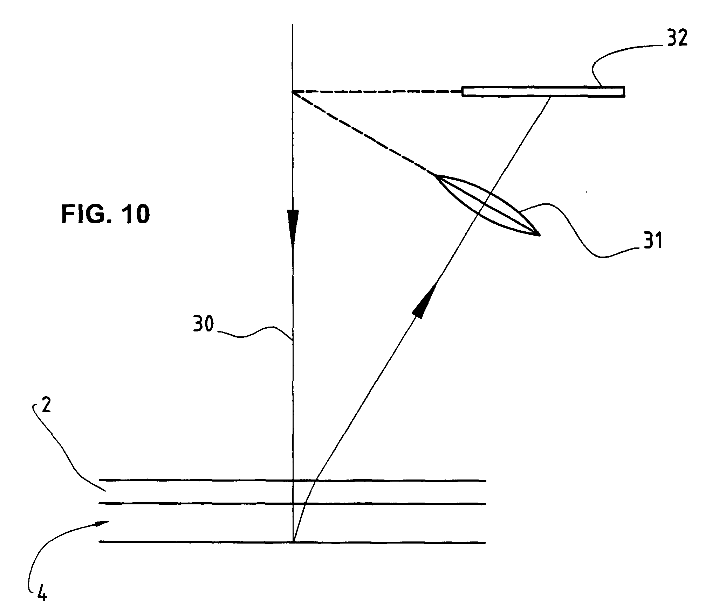

In der Figur 10 wird eine Anordnung für Verfahren basierend auf Lichtstreuung oder Fluoreszenz oder diffuser Reflexion dargestellt, in der der Bildwandler 32 und die Linse 31 der Erfassungsmittel E gemäss der Scheimpflugbedingung angeordnet sind. Dabei muss bei der Auslegung der Empfangsmittel E die durch den Kontaktkörper 2 und die Hornhaut 4 bedingte Brechung der Lichtstrahlen berücksichtigt werden. Im allgemeinen kann damit die Scheimpflugbedingung gut angenähert werden. In der Figur 10 wird beispielhaft ein zum Kontaktkörper 2 senkrecht erzeugter Lichtschnitt 30 über die Linse 31 auf den Bildwandler 32 in Scheimpfluganordnung abgebildet. Bei planparallelem Kontaktkörper 2 kann diese vorteilhafte Konfiguration parallel zur Oberfläche, respektive zur Kontaktfläche des Kontaktkörpers 2 verschoben werden, wobei wegen der fehlenden Hornhautkrümmung keine fortwährende Anpassung der Projektion des Lichtschnitts notwendig ist. Wegen der fehlenden Hornhautkrümmung kann zudem der Tiefenschärfebereich der Lichtquelle 1' und der Erfassungsmittel E verringert und damit die Auflösung erhöht werden. Ein weiterer Vorteil dieser Anordnung ist, dass durch die Richtung der einfallenden Lichtstrahlen streng senkrecht zur Hornhautoberfläche gemessen wird.

Abschliessend soll hier bemerkt werden, dass je nach Ausführungsvariante und gewähltem optischem Verfahren die dazugehörende Lichtquelle 1' und die dazugehörenden Erfassungsmittel E und Verarbeitungsmittel in einer Vorrichtungsanordnung ausgeführt werden, die aus mehreren Einzelkomponenten besteht oder die als kompakte Vorrichtung realisiert ist, die sich insbesondere für den mobilen Einsatz eignet, wenn sie als tragbares Handgerät ausgeführt ist. Durch die vorgeschlagene Verwendung des Kontaktkörpers 2 als Referenz für die Hornhautdickenmessung kann der Messungsaufwand und somit der gerätetechnische Aufwand gegenüber Verfahren des Stands der Technik verringert werden, da zum einen die Hornhaut 4 durch die Kontaktfläche 24, G2 des Kontaktkörpers 2 ausgerichtet wird, das heisst in eine definierte Lage gebracht wird, und zum anderen die Dicke d des Kontaktkörpers 2 zur Messung herangezogen werden kann.

**Patentansprüche**

1.  Verfahren zur Bestimmung der Hornhautdicke (c) eines Auges (3), insbesondere eines menschlichen Auges, mittels Lichtwellen, wobei erste Lichtwellen (L1) auf und in die Hornhaut (4) gestrahlt werden und wobei von der Hornhaut (4) reflektierte zweite Lichtwellen (L3, L4, L7, L8, L10) zur Bestimmung der Hornhautdicke (c) erfasst werden, **dadurch gekennzeichnet,**
    **dass** ein Kontaktkörper (2) als Referenz zur Bestimmung der Hornhautdicke (c) auf mindestens einen Kontaktbereich (B) der Hornhaut (4) aufgesetzt wird, und
    **dass** die ersten Lichtwellen (L1) durch den als Referenz verwendeten Kontaktkörper (2) hindurch auf und in die Hornhaut (4) gestrahlt werden.

2.  Verfahren gemäss Anspruch 1, **dadurch gekennzeichnet, dass** die ersten Lichtwellen (L1) mindestens punktweise in einem zweidimensionalen Messbereich (7) des Kontaktbereichs (B) auf und in die Hornhaut (4) gestrahlt werden, und dass die Hornhautdicke (c) an einer oder mehreren Stellen des Messbereichs (7) bestimmt wird.

3.  Verfahren gemäss einem der Ansprüche 1 oder 2, **dadurch gekennzeichnet, dass** ein Kontaktkörper (2) verwendet wird, der eine vordefinierte Dicke (d) aufweist, und dass die Dicke (d) des Kontaktkörpers (2) zur Bestimmung der Hornhautdicke (c) mitverwendet wird.

4.  Verfahren gemäss einem der Ansprüche 1 bis 3, **dadurch gekennzeichnet, dass** die Hornhaut (4) durch die Kontaktfläche (24) des Kontaktkörpers (2) in eine definierte Lage gebracht wird.

5.  Verfahren gemäss einem der Ansprüche 1 bis 4, **dadurch gekennzeichnet, dass** ein planparalleler Kontaktkörper (2) verwendet wird.

6.  Verfahren gemäss einem der Ansprüche 1 bis 5, **dadurch gekennzeichnet, dass** zusätzlich dritte Lichtwellen (L2, L6, L9), die vom Kontaktkörper (2) reflektiert werden, zur Bestimmung der Hornhautdicke (c) erfasst werden.

7.  Verfahren gemäss einem der Ansprüche 1 bis 6, **dadurch gekennzeichnet, dass** der Kontaktkörper (2) auf der Hornhaut (4) oder dem Auge (3) fixiert wird.

8.  Verfahren gemäss einem der Ansprüche 1 bis 7, **da-**

**durch gekennzeichnet, dass** ein zumindest teilweise semitransparenter Kontaktkörper (2) verwendet wird, der eine Streuung der ersten Lichtwellen (L1) durch Lichtstreuung oder Fluoreszenz im Kontaktkörper (2) oder über diffuse Reflexion an seinen Grenzflächen ermöglicht.

9. Verfahren gemäss einem der Ansprüche 1 bis 8, **dadurch gekennzeichnet, dass** ein Kontaktkörper (2) verwendet wird, dessen Brechungsindex ($n_A$) dem Brechungsindex ($n_B$) der Hornhaut (4) entspricht.

10. Verfahren gemäss einem der Ansprüche 1 bis 9, **dadurch gekennzeichnet, dass** die Lichtquelle (1'), von der die ersten Lichtwellen (L1) ausgestrahlt werden, und/oder die Erfassungsmittel (E), die die reflektierten Lichtwellen (L2, L3, L4, L6, L7, L8, L9, L10) erfassen, gemäss der Scheimpflugbedingung angeordnet werden, um Defokussierungen zu vermeiden.

11. Verfahren gemäss einem der Ansprüche 1 bis 10, **dadurch gekennzeichnet, dass** die ersten Lichtwellen (L1) mit konstantem Einfallswinkel ($\varphi$) auf die Hornhaut (4) gestrahlt werden.

12. Verfahren gemäss einem der Ansprüche 1 bis 11, **dadurch gekennzeichnet, dass** die reflektierten Lichtwellen (L2, L3, L4, L6, L7, L8, L9, L10) unter konstantem Beobachtungswinkel erfasst werden.

13. Verfahren gemäss einem der Ansprüche 1 bis 12, **dadurch gekennzeichnet, dass** die Lichtquelle (1'), von der die ersten Lichtwellen (L1) ausgestrahlt werden, äquidistant zur Kontaktfläche (24) des Kontaktkörpers (2) verschoben wird.

14. Verfahren gemäss einem der Ansprüche 1 bis 13, **dadurch gekennzeichnet, dass** die Erfassungsmittel (E), die die reflektierten Lichtwellen (L2, L3, L4, L6, L7, L8, L9, L10) erfassen, äquidistant zur Kontaktfläche (24) des Kontaktkörpers (2) verschoben werden.

15. Verfahren gemäss einem der Ansprüche 1 bis 14, **dadurch gekennzeichnet, dass** die Lichtquelle (1'), von der die ersten Lichtwellen (L1) ausgestrahlt werden, und die Erfassungsmittel (E), die die reflektierten Lichtwellen (L2, L3, L4, L6, L7, L8, L9, L10) erfassen, mit dem Kontaktkörper (2) verbunden werden.

16. Vorrichtungsanordnung zur Bestimmung der Homhautdicke (c) eines Auges (3), insbesondere eines menschlichen Auges, mittels Lichtwellen, umfassend mindestens eine Lichtquelle (1') zur Strahlung von ersten Lichtwellen (L1) auf und in die Hornhaut (4), Erfassungsmittel (E) zur Erfassung von zweiten Lichtwellen (L3, L4, L7, L8, L10), die von der Hornhaut (4) reflektiert werden, und Verarbeitungsmittel zur Bestimmung der Hornhautdicke (c) aus den erfassten zweiten Lichtwellen (L3, L4, L7, L8, L10), **dadurch gekennzeichnet,**

**dass** die Vorrichtungsanordnung einen Kontaktkörper (2) als Referenz zur Bestimmung der Hornhautdicke (c) umfasst, der auf mindestens einen Kontaktbereich (B) der Hornhaut (4) aufsetzbar ist, und

**dass** die Lichtquelle (1') so angeordnet ist und der Kontaktkörper (2) so beschaffen ist, dass die ersten Lichtwellen (L1) durch den als Referenz verwendeten Kontaktkörper (2) hindurch auf und in die Hornhaut (4) gestrahlt werden.

17. Vorrichtungsanordnung gemäss Anspruch 16, **dadurch gekennzeichnet, dass** sie Mittel (1', 8) umfasst, um die ersten Lichtwellen (L1) mindestens punktweise in einem zweidimensionalen Messbereich (7) des Kontaktbereichs (B), auf und in die Hornhaut (4) zu strahlen, und dass die Erfassungsmittel (E) und die Verarbeitungsmittel so eingerichtet sind, dass die Hornhautdicke (c) an einer oder mehreren Stellen des Messbereichs (7) bestimmt wird.

18. Vorrichtungsanordnung gemäss einem der Ansprüche 16 oder 17, **dadurch gekennzeichnet, dass** der Kontaktkörper (2) eine vordefinierte Dicke (d) aufweist und dass die Erfassungsmittel (E) und die Verarbeitungsmittel so eingerichtet sind, dass die Dicke (d) des Kontaktkörpers (2) zur Bestimmung der Hornhautdicke (c) mitverwendet wird.

19. Vorrichtungsanordnung gemäss einem der Ansprüche 16 bis 18, **dadurch gekennzeichnet, dass** der Kontaktkörper (2) eine Kontaktfläche (24) aufweist, durch die Hornhaut (4) in eine definierte Lage gebracht wird.

20. Vorrichtungsanordnung gemäss einem der Ansprüche 16 bis 19, **dadurch gekennzeichnet, dass** der Kontaktkörper (2) planparallel ist.

21. Vorrichtungsanordnung gemäss einem der Ansprüche 16 bis 20, **dadurch gekennzeichnet, dass** die Erfassungsmittel (E) so eingerichtet sind, dass zusätzlich dritte Lichtwellen (L2, L6, L9), die vom Kontaktkörper (2) reflektiert werden, erfasst werden, und dass die Verarbeitungsmittel so eingerichtet sind, dass die Hornhautdicke (c) unter Berücksichtigung der erfassten dritten Lichtwellen (L2, L6, L9) bestimmt wird.

22. Vorrichtungsanordnung gemäss einem der Ansprüche 16 bis 21, **dadurch gekennzeichnet, dass** sie Mittel (6) umfasst, um den Kontaktkörper (2) auf der Hornhaut (4) oder dem Auge (3) zu fixieren.

23. Vorrichtungsanordnung gemäss einem der Ansprüche 16 bis 22, **dadurch gekennzeichnet, dass** der Kontaktkörper (2) zumindest teilweise semitransparent ist und eine Streuung der ersten Lichtwellen (L1) durch Lichtstreuung oder Fluoreszenz im Kontaktkörper (2) oder über diffuse Reflexion an seinen Grenzflächen ermöglicht.

24. Vorrichtungsanordnung gemäss einem der Ansprüche 16 bis 23, **dadurch gekennzeichnet, dass** der Kontaktkörper (2) einen Brechungsindex ($n_A$) hat, der dem Brechungsindex ($n_B$) der Hornhaut (4) entspricht.

25. Vorrichtungsanordnung gemäss einem der Ansprüche 16 bis 24, **dadurch gekennzeichnet, dass** die Lichtquelle (1') und/oder die Erfassungsmittel (E) gemäss der Scheimpflugbedingung angeordnet sind, um Defokussierungen zu vermeiden.

26. Vorrichtungsanordnung gemäss einem der Ansprüche 16 bis 25, **dadurch gekennzeichnet, dass** sie Mittel umfasst, um die ersten Lichtwellen (L1) mit konstantem Einfallswinkel ($\varphi$) auf die Hornhaut (4) zu strahlen.

27. Vorrichtungsanordnung gemäss einem der Ansprüche 16 bis 26, **dadurch gekennzeichnet, dass** die Erfassungsmittel (E) so eingerichtet sind, dass die reflektierten Lichtwellen (L2, L3, L4, L6, L7, L8, L9, L10) unter konstantem Beobachtungswinkel erfasst werden.

28. Vorrichtungsanordnung gemäss einem der Ansprüche 16 bis 27, **dadurch gekennzeichnet, dass** sie Mittel umfasst, um die Lichtquelle (1') äquidistant zur Kontaktfläche (24) des Kontaktkörpers (2) zu verschieben.

29. Vorrichtungsanordnung gemäss einem der Ansprüche 16 bis 28, **dadurch gekennzeichnet, dass** sie Mittel umfasst, um die Erfassungsmittel (E) äquidistant zur Kontaktfläche (24) des Kontaktkörpers (2) zu verschieben.

30. Vorrichtungsanordnung gemäss einem der Ansprüche 16 bis 29, **dadurch gekennzeichnet, dass** sie Mittel (5) umfasst, um die Lichtquelle (1') und die Erfassungsmittel (E) mit dem Kontaktkörper (2) zu verbinden.

**Claims**

1. Method for determining the corneal thickness (c) of an eye (3), in particular a human eye, by means of light waves, first light waves (L1) being projected onto and into the cornea (4), and second light waves (L3, L4, L7, L8, L10), reflected by the cornea (4), being registered for determining the corneal thickness (c), **characterised**

   **in that** a contact element (2), as a reference for determining the corneal thickness, is placed on at least one contact area (B) of the cornea (4), and

   **in that** the first light waves (L1) are projected through the contact element (2), used as a reference, onto and into the cornea (4).

2. Method according to claim 1, **characterised in that** the first light waves (L1) are projected at least in points into a two-dimensional measuring area (7) of the contact area (B) onto and into the cornea, and the corneal thickness (c) is determined at one or more places of the measuring area (7).

3. Method according to claim 2, **characterised in that** a contact element (2) is used having a predefined thickness (d), and the thickness (d) of the contact element (2) is co-used for determining the corneal thickness (c).

4. Method according to claim 3, **characterised in that** the cornea (4) is brought into a defined state by means of the contact surface (24) of the contact element (2).

5. Method according to claim 3, **characterised in that** a plane-parallel contact element (2) is used.

6. Method according to claim 5, **characterised in that**, additionally, third light waves (L2, L6, L9), which are reflected by the contact element (2), are registered for determining the corneal thickness (c).

7. Method according to one of the claims 1 to 6, **characterised in that** the contact element (2) is fixed on the cornea (4) or on the eye (3).

8. Method according to one of the claims 1 to 6, **characterised in that** an at least partially semi-transparent contact element (2) is used which makes possible a scattering of the first light waves (L1) through light diffusion or fluorescence in the contact element (2) or through diffuse reflection at its boundary surfaces.

9. Method according to one of the claims 1 to 6, **characterised in that** a contact element (2) is used, the refraction index ($n_A$) of which corresponds to the refraction index ($n_B$) of the cornea (4).

10. Method according to one of the claims 1 to 6, **characterised in that** the light source (1'), from which the first light waves (L1) are projected, and/or the registering means (E), which register the reflected light waves (L2, L3, L4, L6, L7, L8, L9, L10), are disposed according to the Scheimpflug condition in order to avoid defocusing.

11. Method according to one of the claims 1 to 6, **characterised in that** the first light waves (L1) are projected with constant incidence angle ($\varphi$) on the cornea (4).

12. Method according to one of the claims 1 to 6, **characterised in that** the reflected light waves (L2, L3, L4, L6, L7, L8, L9, L10) are registered at constant viewing angle.

13. Method according to claim 11, **characterised in that** the light source (1'), from which the first light waves (L1) are projected, is moved at a constant distance from the contact surface (24) of the contact element (2).

14. Method according to one of the claims 1 to 6, **characterised in that** the registering means (E), which register the reflected light waves (L2, L3, L4, L6, L7, L8, L9, L10), are moved at a constant distance from the contact surface (24) of the contact element (2).

15. Method according to one of the claims 1 to 6, **characterised in that** the light source (1'), from which the first light waves (L1) are projected, and the registering means (E), which register the reflected light waves (L2, L3, L4, L6, L7, L8, L9, L10) are connected to the contact element (2).

16. Device configuration for determining the corneal thickness (c) of an eye (3), in particular a human eye, by means of light waves, comprising at least a light source (1') for projection of first light waves (L1) onto and into the cornea (4), registering means (E) for registering second light waves (L3, L4, L7, L8, L10), which are reflected by the cornea (4), and processing means for determining the corneal thickness (c) from the registered second light waves (L3, L4, L7, L8, L10), **characterised in that** the device configuration comprises a contact element (2), as a reference for determining the corneal thickness, which is placeable on at least one contact region (B) of the cornea (4), and **in that** the light source (1') is disposed in such a way and the contact element (2) is designed such that the first light waves (L1) are projected through the contact element (2), used as a reference, onto and into the cornea (4).

17. Device configuration according to claim 16, **characterised in that** it comprises means (1', 8) of projecting the first light waves (L1) at least in points in a two-dimensional measuring area (7) of the contact region (B) onto and into the cornea (4), and the registering means (E) and the processing means are configured in such a way that the corneal thickness (c) is determined at one or more places of the measuring area (7).

18. Device configuration according to claim 17, **characterised in that** the contact element (2) has a predefined thickness (d), and the registering means (E) and the processing means configured in such a way that the thickness (d) of the contact element (2) is co-used for determining the corneal thickness (c).

19. Device configuration according to claim 18, **characterised in that** the contact element (2) has a contact surface (24) by means of which the cornea (4) is brought into a defined state.

20. Device configuration according to claim 18, **characterised in that** the contact element (2) is plane-parallel.

21. Device configuration according to claim 20, **characterised in that** the registering means (E) are configured in such a way that, additionally, third light waves (L2, L6, L9), which are reflected by the contact element (2), are registered, and the processing means is configured in such a way that the corneal thickness (c) is determined taking into account the registered third light waves (L2, L6, L9).

22. Device configuration according to one of the claims 16 to 21, **characterised in that** it comprises means (6) of fixing the contact element (2) on the cornea or on the eye (3).

23. Device configuration according to one of the claims 16 to 21, **characterised in that** the contact element is at least partially semi-transparent and makes possible a scattering of the first light waves (L1) through light diffusion or fluorescence in the contact element (2) or through diffuse reflection at its boundary surfaces.

24. Device configuration according to one of the claims 16 to 21, **characterised in that** the contact element (2) has a refraction index ($n_A$) which corresponds to the refraction index ($n_B$) of the cornea (4).

25. Device configuration according to one of the claims 16 to 21, **characterised in that** the light source (1') and/or the registering means (E) are disposed according to the Scheimpflug condition in order to avoid defocusing.

26. Device configuration according to one of the claims 16 to 21, **characterised in that** it comprises means of projecting the first light source (L1) with constant incidence angle (φ) on the cornea (4).

27. Device configuration according to one of the claims 16 to 21, **characterised in that** the registering means (E) are configured in such a way that the reflected light waves (L2, L3, L4, L6, L7, L8, L9, L10) are registered at a constant viewing angle.

28. Device configuration according to claim 26, **characterised in that** it comprises means of moving the light source (1') at a constant distance from the contact surface (24) of the contact element (2).

29. Device configuration according to one of the claims 16 to 21, **characterised in that** it comprises means of moving the registering means (E) at a constant distance from the contact surface (24) of the contact element (2).

30. Device configuration according to one of the claims 16 to 21, **characterised in that** it comprises means (5) of connecting the light source (1') and the registering means (E) to the contact element (2).

**Revendications**

1. Procédé pour la détermination de l'épaisseur de la cornée (c) d'un oeil (3), en particulier d'un oeil humain au moyen d'ondes lumineuses, les premières ondes lumineuses (L1) étant émises sur et dans la cornée (4) et les secondes ondes lumineuses (L3, L4, L7, L8, L10) réfléchies par la cornée (4) étant saisies pour la détermination de l'épaisseur (c) de la cornée, **caractérisé**

   **en ce qu'**un corps de contact (2) est placé comme référence pour la détermination de l'épaisseur (c) de la cornée sur au moins une zone de contact (B) de la cornée (4) et

   **en ce que** les premières ondes lumineuses (L1) sont émises à travers le corps de contact utilisé comme référence (2) sur et dans la cornée (4).

2. Procédé selon la revendication 1, **caractérisé en ce que** les premières ondes lumineuses (L1) sont émises au moins ponctuellement dans une zone de mesure (7) bidimensionnelle de la zone de contact (B) sur et dans la cornée et **en ce que** l'épaisseur (c) de la cornée est définie sur un ou plusieurs endroits de la zone de mesure (7).

3. Procédé selon l'une des revendications 1 ou 2, **caractérisé en ce que** le corps de contact (2) est utilisé qui présente une épaisseur (d) prédéfinie et **en ce que** l'épaisseur (d) du corps de contact (2) est utilisé pour déterminer l'épaisseur (c) de la cornée.

4. Procédé selon l'une des revendications 1 à 3, **caractérisé en ce que** la cornée (4) est amenée par la surface de contact (24) du corps de contact (2) dans une position définie.

5. Procédé selon l'une des revendications 1 à 4, **caractérisé en ce qu'**un corps de contact parallèle à plan (2) est utilisé.

6. Procédé selon l'une des revendications 1 à 5, **caractérisé en ce qu'**en supplément des troisièmes ondes lumineuses (L2, L6, L9) qui sont réfléchies par des corps de contact (2) sont saisies pour déterminer l'épaisseur (c) de la cornée.

7. Procédé selon l'une des revendications 1 à 6, **caractérisé en ce que** le corps de contact (2) est fixé sur la cornée (4) ou sur l'oeil (3).

8. Procédé selon l'une des revendications 1 à 7, **caractérisé en ce qu'**au moins un corps de contact semi transparent (2) est utilisé qui permet une diffusion des premières ondes lumineuses (L1) par dispersion lumineuse ou fluorescence dans le corps de contact (2) ou par réflexion diffuse à ses surfaces limites.

9. Procédé selon l'une des revendications 1 à 8, **caractérisé en ce qu'**un corps de contact (2) est utilisé dont l'indice de réfraction ($n_A$) correspond à l'indice de réfraction ($n_B$) de la cornée (4).

10. Procédé selon l'une des revendications 1 à 9, **caractérisé en ce que** la source lumineuse (1') depuis laquelle sont émises les premières sources lumineuses (L1) et/ou les moyens de saisie (E) qui saisissent les ondes lumineuses réfléchies (L2, L3, L4, L6, L7, L8, L9, L10) sont disposés selon la condition de Scheimpflug pour éviter des défocalisations.

11. Procédé selon l'une des revendications 1 à 10, **caractérisé en ce que** les premières ondes lumineuses (L1) sont émises sur la cornée (4) avec un angle d'incidence constant (φ).

12. Procédé selon l'une des revendications 1 à 11, **caractérisé en ce que** les ondes réfléchies (L2, L3, L4, L6, L7, L8, L9, L10) sont saisies sous un angle d'observation constant.

13. Procédé selon l'une des revendications 1 à 12, **caractérisé en ce que** la source lumineuse (1') depuis laquelle est émise la première source lumineuse (L1) est déplacée à équidistance de la surface de contact (24) du corps de contact (2).

14. Procédé selon l'une des revendications 1 à 13, **caractérisé en ce que** les moyens de saisie (E) qui saisissent les ondes lumineuses réfléchies (L2, L3, L4, L6, L7, L8, L9, L10) sont déplacés à équidistance de la surface de contact (24) du corps de contact (2).

15. Procédé selon l'une des revendications 1 à 14, **caractérisé en ce que** la source lumineuse (1') depuis laquelle sont émises les premières ondes lumineuses (L1) et les moyens de saisie (E) qui saisissent les ondes lumineuses réfléchies (L2, L3, L4, L6, L7, L8, L9, L10), sont reliées au corps de contact (2).

16. Dispositif pour la détermination de l'épaisseur de la cornée (c) d'un oeil (3) en particulier d'un oeil humain au moyens d'ondes lumineuses comprenant au moins une source lumineuse (1') pour l'émission de premières ondes lumineuses (L1) sur et dans la cornée (4), des moyens de saisie (E) pour la saisie des secondes ondes lumineuses (L3, L4, L7, L8, L10) qui sont réfléchies par la cornée (4) et des moyens de traitement pour la détermination de l'épaisseur (c) de la cornée à partir des secondes ondes lumineuses saisies (L3, L4, L7, L8, L10), **caractérisé en ce que**

le dispositif comprend un corps de contact (2) en tant que référence pour la détermination de l'épaisseur de cornée (c) qui peut être placé sur au moins une zone de contact (B) de la cornée (4) et **en ce que** la source de lumière (1') est disposée et le corps de contact (2) est réalisé de sorte que les premières ondes lumineuses (L1) sont émises à travers le corps de contact (2) utilisé comme référence sur et dans la cornée (4).

17. Dispositif selon la revendication 16, **caractérisé en ce qu'**elle comprend des moyens (1', 8) pour émettre les premières ondes lumineuses (L1) au moins ponctuellement dans une zone de mesure bidimensionnelle (7) de la zone de contacte (B) sur et dans la cornée (4) et **en ce que** les moyens de saisie (E) et les moyens de traitement peuvent être agencés de sorte que l'épaisseur de la cornée (c) est déterminée à un ou plusieurs endroits de la zone de mesure (7).

18. Dispositif selon l'une des revendications 16 ou 17, **caractérisé en ce que** le corps de contact (2) présente une épaisseur prédéfinie (d) et **en ce que** les moyens de saisie (E) et les moyens de traitement sont agencés de sorte que l'épaisseur (d) du corps de contact (2) est utilisée pour la détermination de l'épaisseur de cornée (c).

19. Dispositif selon l'une des revendications 16 à 18, **caractérisé en ce que** le corps de contact (2) présente une surface de contact (24) qui amène la cornée (4) dans une position définie.

20. Dispositif selon l'une des revendications 16 à 18, **caractérisée en ce que** le corps de contact (2) est parallèle à plan.

21. Dispositif selon l'une des revendications 16 à 20, **caractérisé en ce que** les moyens de saisie (E) sont assemblés de sorte qu'en plus des troisièmes ondes lumineuses (L2, L6, L9), qui sont réfléchies par le corps de contact (2), sont saisies et **en ce que** les moyens de traitement sont réalisés de sorte que l'épaisseur de cornée (c) est définie en tenant compte des troisième ondes lumineuses saisies (L2, L6, L9).

22. Dispositif selon l'une des revendications 16 à 21, **caractérisé en ce qu'**il comprend des moyens (6) pour fixer le corps de contact (2) sur la cornée (4) ou sur l'oeil (3).

23. Dispositif selon l'une des revendications 16 à 22, **caractérisé en ce que** le corps de contact (2) est au moins partiellement semi transparent et permet une diffusion des premières ondes lumineuses (L1) par dispersion lumineuse ou fluorescence dans le corps de contact (2) ou par réflexion diffuse à ses surfaces limites.

24. Dispositif selon l'une des revendications 16 à 23, **caractérisée en ce que** le corps de contact (2) présente un indice de réfraction ($n_A$) qui correspond à l'indice de réfraction ($n_B$) de la cornée (4).

25. Dispositif selon l'une des revendications 16 à 24, **caractérisée en ce que** la source lumineuse (1') et/ou les moyens de saisie (E) sont disposés selon les conditions de Scheimpflug pour éviter des défocalisations.

26. Dispositif selon l'une des revendications 16 à 25, **caractérisé en ce qu'**il comprend des moyens pour émettre les premières sources lumineuses (L1) avec un angle d'incidence constant ($\varphi$) sur la cornée (4).

27. Dispositif selon l'une des revendications 16 à 26, **caractérisé en ce que** les moyens de saisie (E) sont agencés de sorte que les ondes lumineuses réfléchies (L2, L3, L4, L6, L7, L8, L9, L10) sont saisies sous un angle d'observation constant.

28. Disposition de dispositifs selon l'une des revendications 16 à 27, **caractérisé en ce qu'**il comprend des moyens pour déplacer la source lumineuse (1') à équidistance de la surface de contact (24) du corps de contact (2).

29. Dispositif selon l'une des revendications 16 à 28, **caractérisé en ce qu'**il comprend des moyens pour déplacer les moyens de saisie (E) à équidistance de la surface de contact (24) du corps de contact (2).

**30.** Dispositif selon l'une des revendications 16 à 29, **caractérisé en ce qu'**il comprend des moyens (5) pour relier la source lumineuse (1') et les moyens de saisie (E) au corps de contact (2).

**FIG. 1**

**FIG. 2**

**FIG. 3**

FIG. 4

FIG. 5

**FIG. 6**

**FIG. 7**

**FIG. 8a**

L1

C

D

24

2

4

B

3

**FIG. 8b**

a''

C

b''

D

20

21

FIG. 9a

FIG. 9b

**FIG. 10**

32

31

30

2

4